# EUROPEAN PATENT APPLICATION

(11) **EP 4 356 905 A1**
(43) Date of publication of application: **24.04.2024**
(21) Application number: 23204473.5
(22) Date of filing: 18.10.2023
(51) Int. Cl.: A61K 9/20, A61K 9/28, A61K 9/48, A61K 31/4439

(54) **TABLETS-IN-CAPSULE COMPRISING LANSOPRAZOLE**

(30) Priority: 19.10.2022 TR 202215882
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to a pharmaceutical composition in the form of capsule having micro or mini tablet wherein; the tablets comprising a core tablet comprising lansoprazole and an inner coating comprising at least one water-soluble polymer and at least one anti-sticking agent and an enteric coating comprising at least one enteric coating polymer. The capsules are obtained by a process which is simple, rapid, cost effective, time-saving and industrially convenient process.

## Description

### Field of the Invention

The present invention relates to a pharmaceutical composition in the form of capsule having micro or mini tablet wherein; the tablets comprising a core tablet comprising lansoprazole and an inner coating comprising at least one water-soluble polymer and at least one anti-sticking agent and an enteric coating comprising at least one enteric coating polymer. The capsules are obtained by a process which is simple, rapid, cost effective, time-saving and industrially convenient process.

### Background of the Invention

Lansoprazole, chemical name 2-(((3-methyl-4-(2,2,2-trifluoroethoxy)-2-pyridyl)methyl) sulfinyl)-1H-benzoyl Imidazole, brownish white crystalline powder, easily soluble in dimethylamide, soluble in methanol, insoluble in ethanol and ether, almost insoluble in water. It has a chemical structure which is shown in the Formula I.

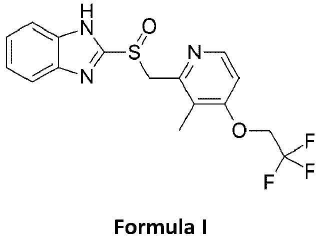

Lansoprazole is the second proton pump inhibitor anti-ulcer drug developed by Takeda Corporation of Japan after omeprazole. 1992ln 1999, Japan's Takeda Pharmaceutical Co., Ltd. and Houde Company first officially put it on the market in France, and in May 1995, it was approved by the FDA to be listed in the United States.

Lansoprazole is extremely unstable under acidic conditions, and is degraded to inactive components in a short time under low pH environment, so it is easily degraded in gastric acid environment. In order to improve the bioavailability of the drug, it needs to be prepared as an enteric-coated preparation.

Chinese Patent Application No.: 201010245689.6 discloses a lansoprazole enteric capsule formulation and preparation method, using sodium bicarbonate as a stabilizer, cross-linked polyvinylpyrrolidone as a disintegrant, and acrylic resin as an enteric coating agent.

U.S. Patent No. 6,328,994 discloses an orally disintegrable lansoprazole tablet which comprises fine enteric coated granules having an average particle diameter of 400µπ 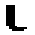 or less.

Therefore, it is still necessary to prepare a pharmaceutical composition in the form of capsule having micro or mini tablet that the desired stability, the desired dissolution profile and is suitable for industrialized production.

### Detailed Description of the Invention

The main object of the present invention is to eliminate problems caused by active substance and bringing additional advantages to the relevant prior art. To explain in more detail, the main purpose is to provide a pharmaceutical composition in the form of capsule having enteric coating micro or mini tablets having high physical and chemical stability and a long shelf life by the help of selection of excipients.

Another object of the present invention is to obtain a pharmaceutical composition in the form of capsule having enteric coating micro or mini tablets providing improved dissolution profile, it also is cost effective.

Another object of the present invention is to obtain a pharmaceutical composition in the form of capsule having enteric coating micro or mini tablets providing improved excellent pharmacomechanic properties, such as flowability, compressibility and content uniformity.

In particular, there is a need to combine an acid-labile physiologically active substance, with basic inorganic salts and so forth for stability, and further to coat with coating layers such as an enteric layer. It is an important problem to produce a small enteric coated micro or mini tablets, even though it contains the acid-labile physiologically active substance in small content. In this invention, a pharmaceutical composition in the form of capsule having micro or mini tablet having both an inner coating and an enteric coating is used. The composition provides the desired stability and the desired profile.

According to one embodiment of the present invention, the tablets in the capsule can be microtablets or minitablets, this varies according to the size of the tablets. The tablets in capsule are cylindrical with a flat or convex upper side and lower side and with a diameter and height which are preferably approximately equal and, independently of one another, preferably have a tablet size of from about 1 mm to about 4 mm, more preferably from about 1.5 mm to about 4.5 mm.

According to one embodiment of the present invention, a pharmaceutical composition in the form of capsule having micro or mini tablet wherein; the tablets comprising
- core tablet comprising lansoprazole,
- inner coating comprising at least one water-soluble polymer and at least one anti-sticking agent,
- enteric coating comprising at least one enteric coating polymer, wherein the weight ratio of the core tablet to the enteric coating is 6.0-1.5.

According to one embodiment of the present invention, the weight ratio of the core tablet to the enteric coating is between 6.0 and 1.5. Preferably it is between 4.0 and 2.0. This ratio provides the desired dissolution profile and, also helps to provide the pharmacomechanic properties.

According to one embodiment of the present invention, the amount of lansoprazole is between 4.0 % and 20.0% by weight in the total composition.

According to one embodiment of the present invention, the amount of core tablet is between 60.0 % and 85.0% by weight in the total composition. Preferably, it is between 65.0 % and 80.0% by weight in the total composition.

According to one embodiment of the present invention, the core tablet comprises lansoprazole, further comprises at least one diluent or at least one binder or at least one disintegrant or at least one lubricant or at least one glidant or at least one surfactant or mixtures thereof.

According to one embodiment of the present invention, the inner coating of the present composition is designed to disintegrate rapidly in an aqueous medium. In the present invention, the inner coating comprises at least one water-soluble polymer and at least one anti-sticking agent. Since it is between the two layers, it is very important for the composition that this layer is effective. Using these excipients in the inner coating provides excellent coating surface for the enteric coating and improves the enteric coating efficiency, while preventing degradations resulting from enteric coating and the tablet core interactions.

Suitable water-soluble polymers are selected from the group comprising polyethylene glycol, polyvinylpyrrolidone, hydroxypropylcellulose, hydroxypropyl methylcellulose or mixtures thereof.

According to one embodiment of the present invention, the water-soluble polymer is hydroxypropyl methylcellulose.

According to one embodiment of the present invention, the amount of water-soluble polymer is between 1.0 % and 7.0% by weight in the total composition. Preferably, the amount of water-soluble polymer is between 1.0 % and 5.0% by weight in the total composition.

Suitable anti-sticking agents are selected from the group comprising talc, starch, kaolin, lactose or dextrose or mixtures thereof.

According to one embodiment of the present invention, the anti-sticking agent is talc.

According to one embodiment of the present invention, the amount of anti-sticking agents is between 0.005 % and 1.0% by weight in the total composition.

According to one embodiment of the present invention, the anti-sticking agents are present in the inner coating or the enteric coating or both.

According to one embodiment of the present invention, the inner coating comprises HPMC as water-soluble polymer and talc as anti-sticking agent. The use of both in the inner coating provided surprisingly high stability, apart from the dissolution profile and ease of manufacture.

According to one embodiment of the present invention, the inner coating further can comprise at least one plasticizer.

Suitable plasticizers are selected from the group comprising triethyl citrate, polyethylene glycol, triacetin, alpha tocopherol, docusate sodium, glyceryl monooleate, glyceryl monostearate, tributyl citrate, acetyl triethyl citrate, acetyl tributyl citrate, butyl stearate, Stearyl alcohol, propylene glycol, diethyl phthalate, dioctyl phosphate, sodium lauryl sulphate, sorbitan esters, potassium cetylphosphate, ethylene glycol distearate, sodium dodecanoate, dioctyl sodium sulfosuccinate, sodium stearate, benzalkonium chlorides, polysorbates, poloxamers or mixtures thereof.

According to one embodiment of the present invention, the plasticizer is triethyl citrate or triacetin or mixtures thereof.

According to one embodiment of the present invention, the plasticizer is triethyl citrate in the enteric coating.

According to one embodiment of the present invention, the plasticizer is triacetin in the inner coating.

According to one embodiment of the present invention, the amount of plasticizers is between 0.5 % and 5.0% by weight in the total composition.

According to one embodiment of the present invention, the plasticizers are present in the inner coating or the enteric coating or both.

According to one embodiment of the present invention, the amount of the inner coating is between 1.0 % and 10.0% by weight in the total composition. Preferably, it is between 1.0 % and 5.0% by weight in the total composition.

According to one embodiment of the present invention, the enteric coating helps to protect lansoprazole from acidic degradation while the dosage form transits the stomach. The enteric coating comprises at least one enteric coating polymer or at least one anti-sticking agent or at least one plasticizer or at least one surfactant or at least one lubricant or at least one pH modifiers or mixtures thereof.

Suitable enteric coating polymers are selected from the group comprising methacrylic acid-methyl methacrylate copolymer (1:2)(Eudragit S100), methacrylic acid-methyl methacrylate copolymer (1:1)(Eudragit L100), hydroxyl propyl methylcellulose (HPMC), glyceryl behenate, hydroxyethyl methylcellulose, hydroxypropyl cellulose, hydroxypropyl ethylcellulose, ethylcellulose, methacrylic acid - ethyl acrylate copolymer, polymethylmetacrylate, polyvinyl acetate, polyvinyl alcohol, polyvinylpyrrolidine, glyceryl dibehenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers or mixtures thereof. It is used only in the enteric coating.

According to one embodiment of the present invention, the enteric coating polymer is methacrylic acid-methyl methacrylate copolymer (1:2)(Eudragit S100) or methacrylic acid-methyl methacrylate copolymer (1:1)(Eudragit L100) or methacrylic acid - ethyl acrylate copolymer (Eudragit L 30 D-55 or Kollicoat MAE 30DP) or mixtures thereof. The methacrylic acid copolymer is used alone or in combination with other enteric polymers to achieve the desired delayed release profile. It also helps to provide stability.

According to one embodiment of the present invention, the amount of enteric coating polymers is between 10.0 % and 25.0% by weight in the total composition.

According to one embodiment of the present invention, the amount of the enteric coating is between 15.0 % and 40.0% by weight in the total composition. Preferably, it is between 20.0 % and 30.0% by weight in the total composition.

Suitable diluents are selected from the group comprising D-mannitol, lactose, microcrystalline cellulose, corn starch, lactose monohydrate, spray-dried mannitol, lactose, starch, dextrose, sucrose, fructose, maltose, sorbitol, xylitol, inorganic salts, calcium salts, polysaccharides, dicalcium phosphate, sodium chloride, maltodextrin, sucrose-maltodextrin mixture, trehalose, magnesium carbonate or mixtures thereof.

According to one embodiment of the present invention, the diluent is D-mannitol or lactose or microcrystalline cellulose or corn starch or magnesium carbonate or mixtures thereof.

According to one embodiment of the present invention, the diluent is D-mannitol.

According to one embodiment of the present invention, the diluent is lactose or microcrystalline cellulose or corn starch or magnesium carbonate or mixtures thereof.

According to one embodiment of the present invention, the amount of diluents is between 25.0 % and 70.0% by weight in the total composition. The amount provides the desired content uniformity. Since the amount of active ingredient is low compared to the whole composition, it is important to ensure content uniformity.

According to one embodiment of the present invention, the diluents are present in core tablet.

Suitable disintegrants are selected from the group comprising croscarmellose sodium, low substituted hydroxypropyl cellulose, crospovidone, ion-exchange resins, magnesium aluminium silica, sodium carboxymethyl cellulose, calcium silicate, sodium starch glycolate, carboxymethyl cellulose, calcium carboxymethyl cellulose, docusate sodium, polyacryline potassium, poloxamer, povidone, sodium alginate, sodium glycine carbonate, or mixtures thereof.

According to one embodiment of the present invention, the disintegrant is croscarmellose sodium or low substituted hydroxypropyl cellulose or crospovidone mixtures thereof.

According to one embodiment of the present invention, the amount of disintegrant is between 1.0 % and 15.0% by weight in the total composition.

According to one embodiment of the present invention, the disintegrants are present in the core tablet.

Suitable binders are selected from the group comprising hydroxypropyl cellulose, polyvinylpyrrolidone, natural gums, gelatin, alginates, sodium alginate, gum, guar gum, starch mucilage, acacia mucilage, cetostearyl alcohol, pullulan, polydextrose, polyethylene oxide, pectin, aluminum hydroxide, hyaluronic acid or mixtures thereof.

According to one embodiment of the present invention, the binder is hydroxypropyl cellulose or polyvinylpyrrolidone or mixtures thereof.

According to one embodiment of the present invention, the binders are present in core tablet.

According to one embodiment of the present invention, the amount of binder is between 0.5 % and 15.0% by weight in the total composition.

Suitable glidants are selected from the group comprising talc, colloidal silica anhydrous, colloidal silicon dioxide, calcium silicate, aluminium silicate, magnesium silicate, magnesium oxide, starch or mixtures thereof.

According to one embodiment of the present invention, the glidant is talc or colloidal silica anhydrous or mixtures thereof.

According to one embodiment of the present invention, when talc is used in the core tablet, it acts as a glidant. When talc is used in the inner coating or enteric coating, it acts as an anti-adhesive agent.

According to one embodiment of the present invention, the glidants are present in the core tablet.

According to one embodiment of the present invention, the amount of glidant is between 0.1 % and 4.0% by weight in the total composition.

Suitable surfactants are selected from the group comprising polysorbate 80, glyceryl monostearate, sodium dodecyl sulphate, sodium lauryl sulfate, cetylpyridinium chloride, docusate sodium, lauric acid, polyoxyethylene sorbitan fatty acid esters (polysorbate), phospholipids, cetrimide or mixtures thereof.

According to one embodiment of the present invention, the surfactant is polysorbate 80 or glyceryl monostearate or sodium lauryl sulfate or mixtures thereof. It is a synthetic organic compound widely used in pharmaceutical products as an anionic surfactant. In the present invention, it provides the desired profile. It also helps to provide flowability.

According to one embodiment of the present invention, the amount of surfactant is between 0.1 % and 5.0% by weight in the total composition.

According to one embodiment of the present invention, the surfactants are present in the core tablet or the enteric coating or both.

Suitable pH modifiers are selected from the group comprising aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof. Using Ph modifier ensures that lansoprazole, which is unstable at low pH, remains at the desired stability under long conditions.

According to one embodiment of the present invention, the pH modifier is sodium bicarbonate.

According to one embodiment of the present invention, the pH modifier is present in the enteric coating.

Suitable lubricants are selected from the group comprising magnesium stearate, sodium stearyl fumarate, calcium stearate, stearic acid, polyethylene glycol, sodium lauryl sulfate, magnesium lauryl sulfate, fumaric acid, glyceryl palmito sulfate, hydrogenated vegetable oil, zinc stearate or mixtures thereof.

According to one embodiment of the present invention, the lubricant is magnesium stearate or sodium stearyl fumarate or mixtures thereof.

According to one embodiment of the present invention, the lubricants are present in the core tablet or the enteric coating or both.

According to one embodiment of the present invention, the amount of lubricant is between 1.0 % and 7.0% by weight in the total composition.

According to one embodiment of the present invention, the core tablet comprises lansoprazole, D-mannitol, Low substituted HPC, sodium stearyl fumarate, croscarmellose sodium and hydroxypropyl cellulose.

According to one embodiment of the present invention, the core tablet comprises lansoprazole, microcrystalline cellulose, magnesium carbonate and magnesium stearate.

According to one embodiment of the present invention, the inner coating comprises HPMC, triacetin and talc.

According to one embodiment of the present invention, the inner coating comprises HPMC and talc.

According to one embodiment of the present invention, the enteric coating comprises metacrylic acid ethyl acrylate copolymer (1.1) type A, talc, triethyl citrate, colloidal silica anhydrous, SLS and sodium bicarbonate.

According to one embodiment of the present invention, the enteric coating comprises Eudragit S100, Eudragit L100, talc, triethyl citrate, glyceryl monostearate and Polysorbate 80.

### Example 1:

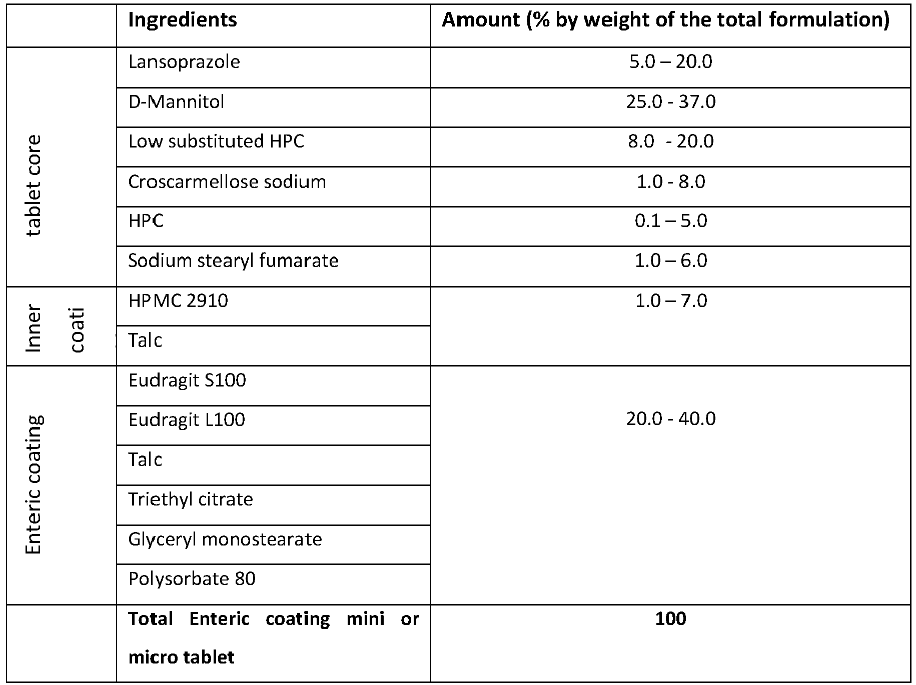

A process for example 1;
Core tablet
   - Weighing Lansoprazole, D-mannitol, HPC, croscarmellose sodium, low substituted HPC and mixing for 15 minutes at roller compact (roller gap 2.0 mm, roller pressure 15.0 mPa, feed screw speed of 10:1, feed screw speed 25 rpm, speed 2.5 rpm)
   - Sieving the granules,
   - Adding sodium stearyl fumarate to dry mixture and mix for 5 minutes,
   - Compressing the mixture into the tablet,
inner coating
   - Performing enteric coating with HPMC and talc,
Enteric coating
   - Performing enteric coating with Methacrylic acid copolymer dispersion,
Filling the micro or mini tablets having inner and enteric coating into capsules.

### Example 2:

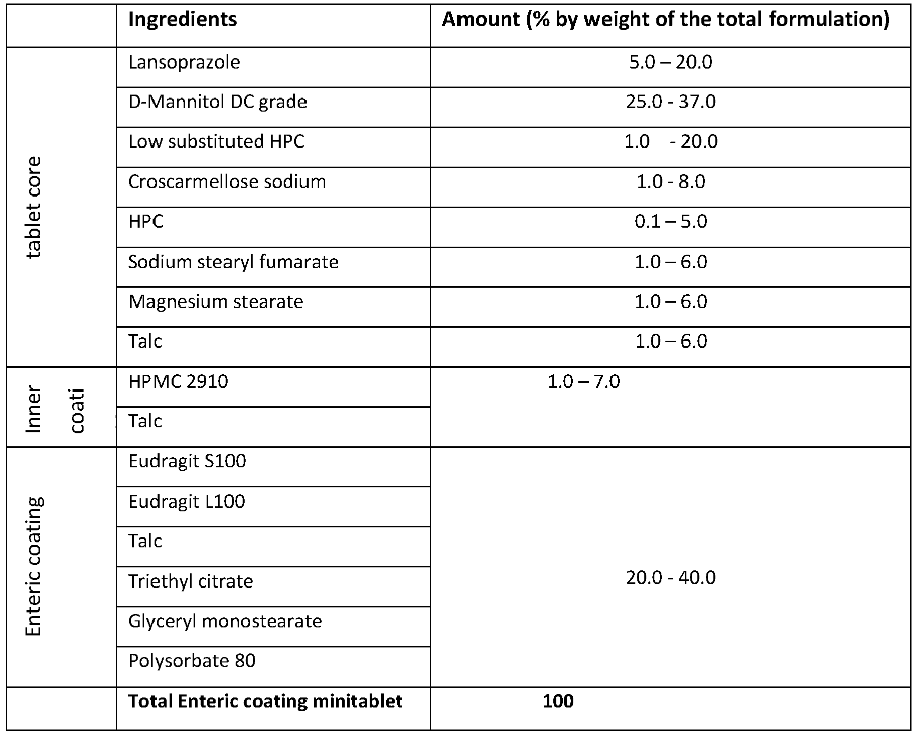

A process for example 2;
Core tablet
   - Weighing Lansoprazole, D-mannitol, HPC, croscarmellose sodium, low substituted HPC and mixing for 15 minutes,
   - Adding sodium stearyl fumarate and talc and then mixing for 5 minutes,
   - Adding Magnesium stearate to dry mixture and mix for 3 minutes,
   - Compressing the mixture into the tablet,
inner coating
   - Performing enteric coating with HPMC and talc,
Enteric coating
   - Performing enteric coating with Methacrylic acid copolymer dispersion,
Filling the micro or mini tablets having inner and enteric coating into capsules.

### Example 3:

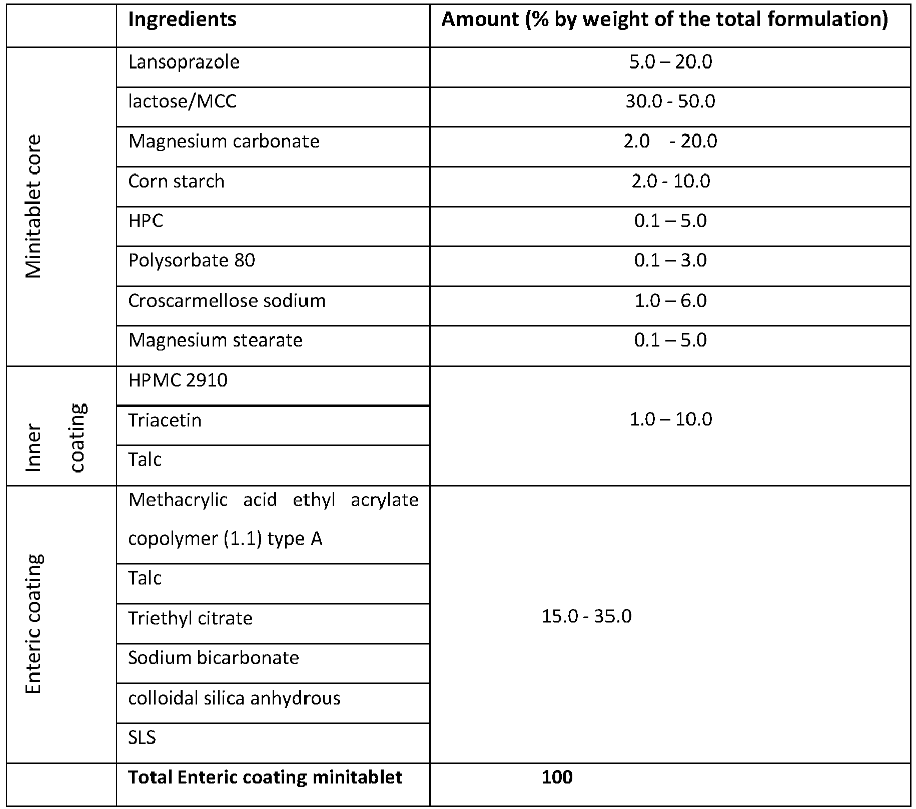

A process for example 3;
Core tablet
   - Weighing Lansoprazole, magnesium carbonate, lactose, ½ croscarmellose sodium, and mixing for 15 minutes,
   - Granulating the dry mixture with HPC and polysorbate 80 in water solution,
   - Sieving 2.0 mm wet granules,
   - Drying the granules in oven,
   - Sieving dry granules into 0.85mm,
   - Adding ½ croscarmellose sodium, and mixing for 5 minutes,
   - Adding weighed and 0.6 mm sieved Magnesium stearate to dry mixture and mix for 3 minutes,
   - Compressing the mixture into the tablet,
inner coating
   - Performing enteric coating with HPMC, triacetin and talc,
Enteric coating
   - Performing enteric coating with Methacrylic acid copolymer dispersion, Filling the micro or mini tablets having inner and enteric coating into capsules.

### Example 4:

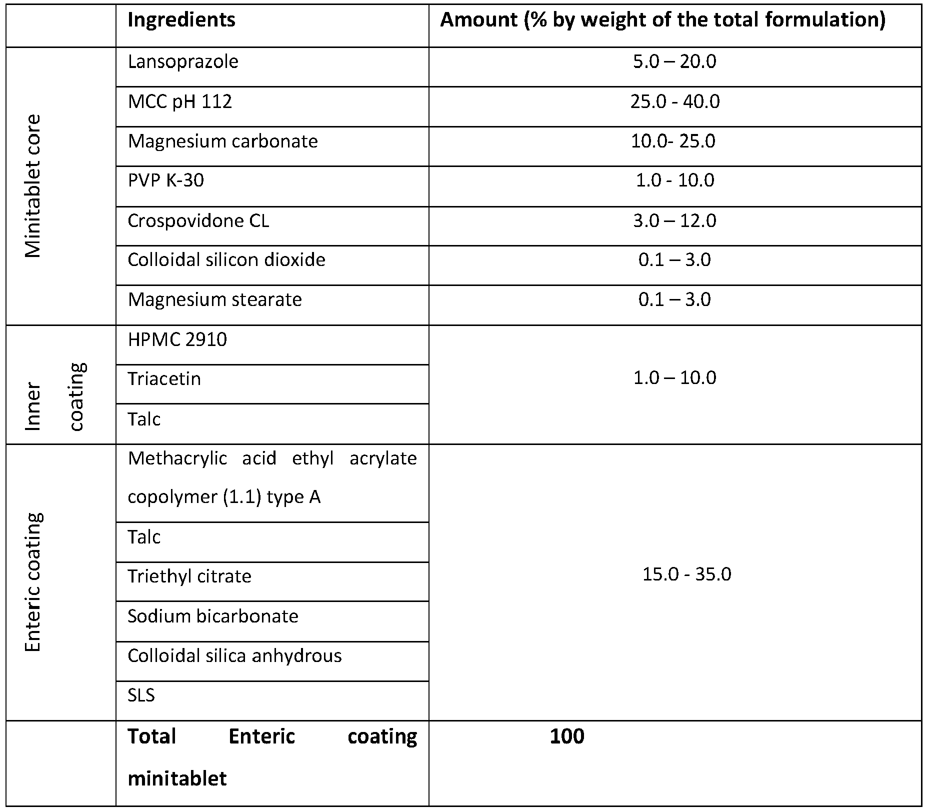

A process for example 4;
Core tablet
   - Weighing Lansoprazole, Magnesium carbonate, ½ MCC 112, colloidal silicon dioxide, PVP K-30 and mixing for 10 minutes,
   - Adding weighed and 0.6 mm sieved ½ Magnesium stearate to dry mixture and mix for 3 minutes,
   - Performing slug granulation in compactor,
   - Sieving dry granules 1.2 mm,
   - Adding ½ MCC pH 112, Crospovidone CL and mix for 5 minutes
   - Adding 0.6mm sieved ½ Mg-stearate and mix for 3 minutes
   - Compressing the mixture into the tablet,
inner coating
   - Performing enteric coating with HPMC, triacetin and talc,
Enteric coating
   - Performing enteric coating with Methacrylic acid copolymer dispersion, Filling the micro or mini tablets having inner and enteric coating into capsules.

### Example 5:

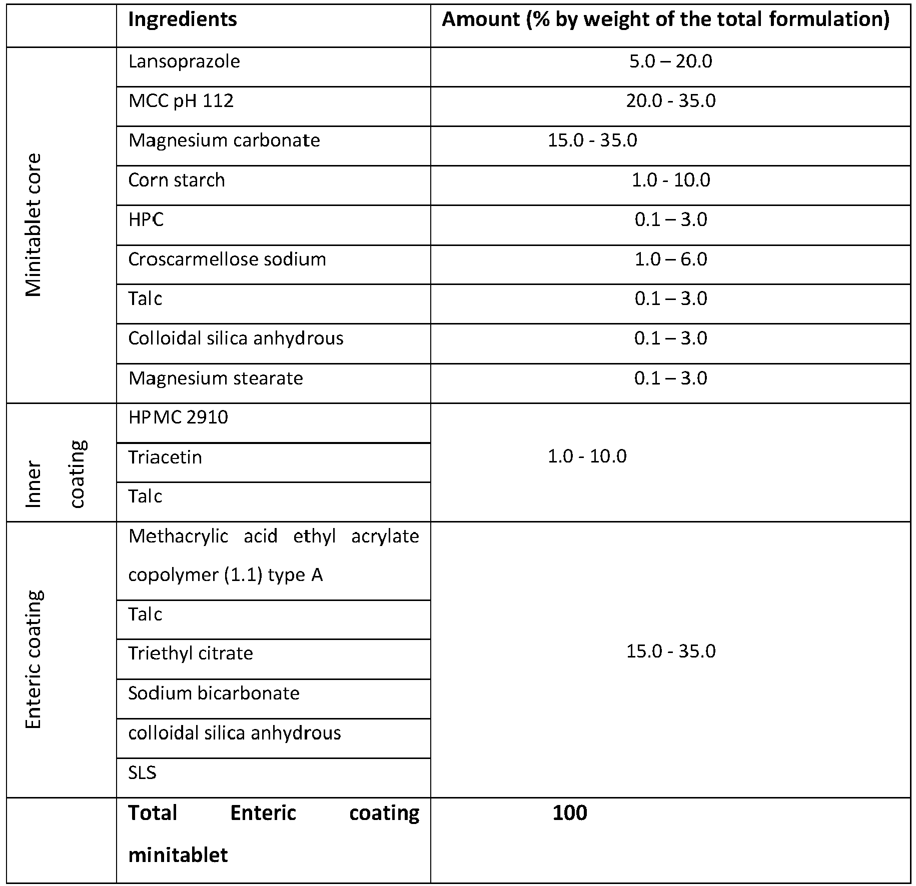

A process for example 5;
Core tablet
   - Weighing Lansoprazole, Magnesium carbonate, MCC 112, HPC, corn starch, croscarmellose, sodium, colloidal silica anhydrous, talc and mixing for 15 minutes,
   - Adding weighed and 0.6 mm sieved Magnesium stearate to dry mixture and mix for 3 minutes,
   - Compressing the mixture into the tablet,
inner coating
   - Performing enteric coating with HPMC, triacetin and talc,
Enteric coating
   - Performing enteric coating with Methacrylic acid copolymer dispersion,

Filling the micro or mini tablets having inner and enteric coating into capsules.

## Claims

1. A pharmaceutical composition in the form of capsule having micro or mini tablet wherein; the tablets comprising
- core tablet comprising lansoprazole,
- inner coating comprising at least one water-soluble polymer and at least one anti-sticking agent,
- enteric coating comprising at least one enteric coating polymer,
Wherein the weight ratio of the core tablet to the enteric coating is 6.0-1.5.

2. The pharmaceutical composition in the form of capsule having micro or mini tablet according to claim 1, wherein the core tablet comprises lansoprazole, further comprises at least one diluent or at least one binder or at least one disintegrant or at least one lubricant or at least one glidant or at least one surfactant or mixtures thereof.

3. The pharmaceutical composition in the form of capsule having micro or mini tablet according to claim 1, wherein water-soluble polymers are selected from the group comprising polyethylene glycol, polyvinylpyrrolidone, hydroxypropylcellulose, hydroxypropyl methylcellulose or mixtures thereof.

4. The pharmaceutical composition in the form of capsule having micro or mini tablet according to claim 1, wherein anti-sticking agents are selected from the group comprising talc, starch, kaolin, lactose or dextrose or mixtures thereof.

5. The pharmaceutical composition in the form of capsule having micro or mini tablet according to claim 3 or 4, wherein the inner coating comprises HPMC as water-soluble polymer and talc as anti-sticking agent.

6. The pharmaceutical composition in the form of capsule having micro or mini tablet according to claim 1, wherein the enteric coating comprises at least one enteric coating polymer or at least one anti-sticking agent or at least one plasticizer or at least one surfactant or at least one lubricant or at least one pH modifiers or mixtures thereof.

7. The pharmaceutical composition in the form of capsule having micro or mini tablet according to claim 5, wherein the inner coating further can comprise at least one plasticizer.

8. The pharmaceutical composition in the form of capsule having micro or mini tablet according to claim 5 or 6, wherein plasticizers are selected from the group comprising triethyl citrate, polyethylene glycol, triacetin, alpha tocopherol, docusate sodium, glyceryl monooleate, glyceryl monostearate, tributyl citrate, acetyl triethyl citrate, acetyl tributyl citrate, butyl stearate, Stearyl alcohol, propylene glycol, diethyl phthalate, dioctyl phosphate, sodium lauryl sulphate, sorbitan esters, potassium cetylphosphate, ethylene glycol distearate, sodium dodecanoate, dioctyl sodium sulfosuccinate, sodium stearate, benzalkonium chlorides, polysorbates, poloxamers or mixtures thereof.

9. The pharmaceutical composition in the form of capsule having micro or mini tablet according to claim 8, wherein the plasticizer is triethyl citrate or triacetin or mixtures thereof.

10. The pharmaceutical composition in the form of capsule having micro or mini tablet according to claim 6, wherein enteric coating polymers are selected from the group comprising methacrylic acid-methyl methacrylate copolymer (1:2)(Eudragit S100), methacrylic acid-methyl methacrylate copolymer (1:1)(Eudragit L100), hydroxyl propyl methylcellulose (HPMC), glyceryl behenate, hydroxyethyl methylcellulose, hydroxypropyl cellulose, hydroxypropyl ethylcellulose, ethylcellulose, methacrylic acid - ethyl acrylate copolymer, polymethylmetacrylate, polyvinyl acetate, polyvinyl alcohol, polyvinylpyrrolidine, glyceryl dibehenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers or mixtures thereof. It is used only in the enteric coating.

11. The pharmaceutical composition in the form of capsule having micro or mini tablet according to claim 10, wherein the enteric coating polymer is methacrylic acid-methyl methacrylate copolymer (1:2)(Eudragit S100) or methacrylic acid-methyl methacrylate copolymer (1:1)(Eudragit L100) or methacrylic acid - ethyl acrylate copolymer or mixtures thereof.

12. The pharmaceutical composition in the form of capsule having micro or mini tablet according to claim 2, wherein diluents are selected from the group comprising D-mannitol, lactose, microcrystalline cellulose, corn starch, lactose monohydrate, spray-dried mannitol, lactose, starch, dextrose, sucrose, fructose, maltose, sorbitol, xylitol, inorganic salts, calcium salts, polysaccharides, dicalcium phosphate, sodium chloride, maltodextrin, sucrose-maltodextrin mixture, trehalose, magnesium carbonate or mixtures thereof.

13. The pharmaceutical composition in the form of capsule having micro or mini tablet according to claim 12, wherein the diluent is D-mannitol or lactose or microcrystalline cellulose or corn starch or magnesium carbonate or mixtures thereof.

14. The pharmaceutical composition in the form of capsule having micro or mini tablet according to claim 13, wherein wherein the amount of diluents is between 25.0 % and 70.0% by weight in the total composition.

15. The pharmaceutical composition in the form of capsule having micro or mini tablet according to claim 2 or 6, wherein surfactants are selected from the group comprising polysorbate 80, glyceryl monostearate, sodium dodecyl sulphate, sodium lauryl sulfate, cetylpyridinium chloride, docusate sodium, lauric acid, polyoxyethylene sorbitan fatty acid esters (polysorbate), phospholipids, cetrimide or mixtures thereof.
